(19) Europäisches Patentamt
European Patent Office
Office européen des brevets

(11) Veröffentlichungsnummer: **0 052 075**
**A1**

# (12) EUROPÄISCHE PATENTANMELDUNG

(21) Anmeldenummer: **81810443.2**

(22) Anmeldetag: **06.11.81**

(51) Int. Cl.³: **A 61 K 9/32**, A 61 K 9/36, A 61 K 9/52

(30) Priorität: **12.11.80 CH 8392/80**

(43) Veröffentlichungstag der Anmeldung: **19.05.82**
**Patentblatt 82/20**

(84) Benannte Vertragsstaaten: **AT BE CH DE FR IT LI LU NL SE**

(71) Anmelder: **CIBA-GEIGY AG, Patentabteilung Postfach, CH-4002 Basel (CH)**

(72) Erfinder: **Kopf, Helmut, Dr., Waldhofstrasse 10, CH-4310 Rheinfelden (CH)**

(54) **Körnige Arzneimittel-Retardform.**

(57) Körnige Arzneimittel-Retardform, enthaltend einen granulierten oder kristallinen Arzneimittelwirkstoff, umhüllt mit wirkstoffabgabeverzögernden Hüllstoffen, wobei diese Hüllstoffe im wesentlichen aus einem homogenen Gemisch eines wasserunlöslichen, aber in Wasser dispergierbaren Polyacrylsäureesters und eines wasserunlöslichen aber im Wasser dispergierbaren Celluloseäthers bestehen.

EP 0 052 075 A1

Körnige Arzneimittel-Retardform

Die Erfindung betrifft eine neue körnige Arzneimittel-Retardform.

Es ist bekannt, Arzneimittelwirkstoffe mit Wirkstoffabgabe-verzögernden Hüllstoffen zu vermischen bzw. zu umhüllen und zu Körnern zu verarbeiten, die dann in dieser körnigen Form direkt verabreicht werden können, oder nach dem Abfüllen in Kapseln oder erst nach Weiterverarbeitung zu Tabletten, zur Therapie verwendet werden. Bisher bekannte gekörnte Retard-Arzneimittel hatten mannigfache Nachteile. Es ergaben sich Schwierigkeiten im Zusammenhang mit der Herstellung. Entweder war die Herstellungsweise umständlich oder es war die Verwendung von organischen Lösungsmitteln notwendig oder es waren die Hilfsstoffe nicht ideal im Hinblick auf den anvisierten Effekt, nämlich die richtig verzögerte Wirkstoffabgabe. Schwierigkeiten ergaben sich auch mit den äussern Eigenschaften dieser Körner, also z.B. mit der Freifliess-Fähigkeit oder der Feuchtigkeitsempfindlichkeit, Eigenschaften, die entweder bei der direkten Verabreichung, also bei der Dosierung und eventuell bei gleichzeitiger Einnahme mit Nahrungs- oder Genussmitteln oder bei der Abfüllung in Kapseln unangenehm in Erscheinung traten.

Die erfindungsgemässe körnige Arzneimittel-Retardform, die einen granulierten oder kristallinen Arzneimittelwirkstoff enthält, der umhüllt ist mit Wirkstoffabgabe-verzögernden Hüllstoffen, ist dadurch gekennzeichnet, dass diese Hüllstoffe im wesentlichen aus einem homogenen Gemisch eines wasserunlöslichen, aber in Wasser dispergierbaren Polyacrylsäureesters und eines wasserunlöslichen, aber in Wasser

dispergierbaren Celluloseäthers bestehen.

Die beiden erfindungsgemäss verwendeten Hüllstoffe sind einzeln als solche bekannt. Sie eignen sich allein angewandt für die vorliegende Erfindung aber nicht. Der erstgenannte ist sehr thermoplastisch und damit hergestellte umhüllte Körner neigen zum Verkleben. Der zweitgenannte andererseits ergibt in gebräuchlichen Mengen und Verarbeitungsverfahren bei der vorliegenden Anwendung eine zu wenig retardierende Umhüllung. Es konnte somit nicht erwartet werden, dass die Kombination der für den vorliegenden Zweck einzeln nicht geeigneten Hüllstoffe ein in jeder Beziehung sehr gutes Resultat ergibt. So sind die erfindungsgemäss hergestellten Arzneimittelkörner freifliessend, unempfindlich gegen Feuchtigkeit, geschmacksneutral und sie erzielen die gewünschte verzögerte Wirkstoffabgabe mit grosser Gleichmässigkeit. Durch mikroskopische Untersuchung wurde ausserdem festgestellt, dass das einzelne Wirkstoffkorn sehr gleichmässig überzogen ist, so dass es im wesentlichen seine ursprüngliche Form beibehält. Somit kann mit homogenem Ausgangsmaterial leicht ein homogenes Endprodukt erzielt werden. Ein weiterer Vorteil der Erfindung besteht darin, dass die Hüllstoffe bei der Herstellung der erfindungsgemässen Produkte als wässerige Dispersion verwendet werden, was gegenüber sonst gebräuchlichen Hüllstoffen, die in organischen Lösungsmitteln dispergiert werden müssen, erhöhte Sicherheit und geringere Umweltbelastung mit sich bringt.

Als Wirkstoffe für die erfindungsgemässe körnige Arzneimittel-Retardform eignen sich besonders körnige oder kristalline Stoffe. Besonders geeignet sind feste Körner oder Monokristalle im Grössenbereich 0,3 - 2 mm (Durchmesser), die eine gewisse mechanische Festigkeit aufweisen, was von besonderer Bedeutung ist, wenn die überzogenen Körner anschliessend zu Presslingen verarbeitet werden.

Als Hüllstoffe eignen sich besonders einerseits Polyacrylsäureester der Formel

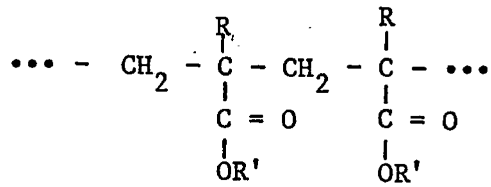

R = H, $CH_3$

R' = $CH_3$, $C_2H_5$

Solche Stoffe werden durch Emulsionspolymerisation gewonnen und enthalten das Copolymerisat mit einem Molgewicht von einigen 100 000 in Form von Latexteilchen mit einem Durchmesser um oder unter 1 µm. Ein entsprechendes besonders geeignetes Produkt wird durch Röhm Pharma GmbH, Darmstadt (BRD) unter der Bezeichnung Eudragit® E30D als wässrige Dispersion vertrieben, und stellt ein Acrylsäureäthylester-Methacrylsäuremethylester 70:30-Copolymerisat, mit Mol-Gew. 800 000 dar.

Andererseits wird als Hüllstoff vorzugsweise Aethylcellulose verwendet. Besonders eignet sich ein Produkt, das von FMC Corporation, Philadelphia (Pennsylvania, USA) unter der Bezeichnung Aquacoat® ECD-30 vertrieben wird, und zwar als 30 %-ige wässrige polymere Dispersion mit geringer Teilchengrösse (Latexform) und enger Teilchengrössenverteilung.

Die beiden oben genannten Hüllstoffe [Poly(H+meth)-acrylsäure-(methyl + äthyl)ester und Aethylcellulose] werden vorzugsweise im Gewichtsverhältnis 2,5:1 bis 5:1, aber insbesondere im Verhältnis 3:1 verwendet.

Es ist vorteilhaft, den erfindungsgemäss umhüllten Körnern geringe Mengen, z.B. von 0,5 bis 1 %, kolloidales Siliziumdioxid, z.B. das durch Degussa, Frankfurt (BRD) vertriebene Aerosil®, zuzumischen. Durch diesen Zusatz wird die Freifliessfähigkeit der Körner noch verbessert.

Weiter können natürlich dem Hüllstoffgemisch auch sonstige Hilfsstoffe in geringen Mengen, wie z.B. Farbstoffe oder Aromastoffe zugesetzt werden.

Die erfindungsgemäss hergestellten Arzneimittelkörner können als solche in den Handel gebracht werden, zur individuellen Dosierung und gegebenenfalls zur Einarbeitung in Nahrungs- oder Genussmittel. Die Handelsform wäre dann in üblichen Feststoffbehältern enthalten, und zwar mit oder ohne Dosiervorrichtung. Zur vordosierten Abgabe der Arzneimittelkörner eignen sich auch Kapseln.

Die Herstellung der erfindungsgemässen Arzneimittelkörner kann in an sich bekannter Weise erfolgen, also in den für diesen Zweck bekannten Wirbelschicht-Sprühapparaten oder in Dragierkesseln. Das Hüllstoffgemisch wird als wässrige Dispersion bei Raumtemperatur zugeführt. Das Versprühen erfolgt am besten mit Luft von einer Temperatur von 25 bis 30°C. Es kann nach dem Gleichstrom- oder Gegenstromprinzip gearbeitet werden; bevorzugt wird aber das erstere. Auf diese Weise werde ohne weiteres die einzelnen Körner erhalten, d.h. eine unerwünschte Agglomeration zu Granulaten erfolgt nicht.

Ueberraschenderweise lassen sich die erfindungsgemäss hergestellten Arzneimittelkörner zusammen mit einem Sprengmittel mit hohen Sprengstoff- und Bindemitteleigenschaften und mit den sonst für die Tablettierung üblichen Hilfsstoffen innerhalb eines weiten Dosisbereiches leicht zu Formkörpern, wie z.B. Tabletten oder kapsel- oder stäbenchförmigen Presslingen verpressen. Die so hergestellten Formkörper haben die Eigenschaften, dass sie bereits schon im Magen des so Behandelten rasch wieder in Einzelkörner zerfallen und sich somit gut verteilen. Auf diese Weise wird eine örtliche Ueberkonzentration des Wirkstoffes im Verdauungstrakt verhindert und für eine gleichmässige,

langsam und über ein grosses Resoptionsgebiet verteilte Wirkstoffabgabe gesorgt. Durch mikroskopische Untersuchung wurde festgestellt, dass die einzelnen Körner durch das Verpressen kaum beschädigt werden, so dass bei der Freisetzung des Wirkstoffes aus denselben diese ihre ursprünglichen vorteilhaften Eigenschaften praktisch voll entfalten können. Soll ein Pressling mit zwei oder mehreren Wirkstoffen hergestellt werden, so können individuelle gefärbte Arzneistoffkörner separat hergestellt werden, was die Kennzeichnung verbessert und dann auch den Patienten darauf aufmerksam macht, dass er ein Medikament mit zwei oder mehreren Wirkstoffen vor sich hat.

Als Sprengmittel mit Bindemitteleigenschaften für die erfindungsgemäss herstellbaren Presslinge eignen sich insbesondere quervernetztes Polyvinylpolypyrrolidon (PVPP), wie z.B. das durch die GAF Corporation, New York, N.Y. (USA) vertriebene Polyplasdone® XL bzw. Kollidon® CL (BASF, Ludwigshafen/Rhein, BRD) oder Natriumcarboxymethylstärken, wie z.B. die durch W.A. Scholten's Chemische Fabriken N.V. Foxhol (NL) vertriebene Primojel® oder der durch E. Mendell Co. Inc., New York (USA) vertriebene Explotab®.

Bei den sonst noch für die Tablettierung üblichen Hilfsstoffen handelt es sich vor allem um Binde- und Schmier- bzw. Antiklebemittel.

Zur Herstellung der erfindungsgemäss herstellbaren Presslinge können die üblichen Tablettenpressen verwendet werden.

Da die mechanische Festigkeit der Presslinge überraschend gut ist, lassen sich auch alle gewünschten üblichen Formen, wie z.B. Tabletten, kapsel- oder stäbchen-förmige Presslinge mit oder ohne Bruchkerben herstellen. Diese Presslinge lassen sich gewünschtenfalls auch mit einer für diesen Zweck bekannten Schutzlackierung versehen.

Als erfindungsgemäss zu verarbeitende Arzneimittelwirkstoffe von Granulatform bzw. Kristallen geeigneter Grösse eignen sich im Prinzip alle, die für eine perorale Abgabe geeignet sind und für die eine verzögerte Abgabe im Magen-Darmtrakt erwünscht ist. Besonders vorteilhaft ist die vorliegende Erfindung jedoch bei Verwendung von Wirkstoffen,die bei höherer Konzentration lokale Reizungen der Schleimhäute im Magen-Darmtrakt verursachen können, und die in grossen Einzeldosen verabreicht werden.Dies trifft z.B. für Kaliumchlorid zu, das bei Kaliummangelzuständen verabreicht wird, oder für Lithiumsalze in der Psychotherapie.

**Beispiel 1**

**Zusammensetzung:**

| | pro Dosis | pro Ansatz |
|---|---|---|
| Kaliumchloridkristalle mit Teilchengrössen von 0,5-1,2 mm | 600,0 mg | 1200,0 g |
| Eudragit® E30D Feststoff | 108,0 mg | 216,0 g (720 g Dispersion) |
| Aquacoat® ECD 30 Feststoff | 33,0 mg | 66,0 g (220 g Dispersion) |
| Aerosil® 200 | 4,0 mg | 8,0 g |
| | 745,0 mg | 1490,0 g |

**Herstellung:**

1. Kaliumchlorid vorlegen.
2. Eudragit® E30D und Aquacoat® ECD30 unter schwachem Rühren mischen.
3. 1. mit 2. besprühen im Wirbelschichtgranulator (Wirbelschichtgranulator Aeromatik ST 7):
   - im Gleichstrom sprühen
   - die Mischung der beiden Dispersionen während des Sprühvorganges rühren
   - Zulufttemperatur 28°C
   - Durchsatz ca. 8g/Minute

4. Trocknen bei 28°C Zulufttemperatur (Wirbelschichttrockner Aeromatik ST 7) ca. 10 Minuten.
5. Trockene umhüllte Körner mit Aerosil® 200 während 10 Minuten mischen.
6. Mischung 5. sieben, Sieb 1.5-2.0 mm.

Eigenschaften

Freifliessend, geschmacklos.

Freigabeeigenschaften von Kaliumchlorid (Vanderkamp Zerfallstester, in Wasser von 37°C):

| | |
|---|---|
| nach 1 Stunde | ca. 24 % |
| nach 2 Stunden | ca. 45 % |
| nach 4 Stunden | ca. 81 % |
| nach 6 Stunden | ca. 96 % |

Abfüllung in Gelatinekapseln:

Die nach Beispiel 1 hergestellten Körner werden mittels einer Höfliger und Karg Kapselfüllmaschine GKF 330 mit Körnerdosiervorrichtung (Dosierkammerformat 15 mm, 85 UpM) in Gelatinekapseln (Snap Fit® [Capsugel AG] oder Lok Caps® [Elanco] Grösse 0 abgefüllt.

Beispiel 2:

Zusammensetzung:

| | pro Dosis | pro Ansatz |
|---|---|---|
| Kaliumchloridkristalle mit Teilchengrössen von 0,3 - 0,8 mm | 600,0 mg | 900,0 g |
| Eudragit® E30D Feststoff | 115,0 mg | 172,5 g (575 g Dispersion) |
| Aquacoat® ECD 30 Feststoff | 35,0 mg | 52,5 g (175 g Dispersion) |
| Aerosil® 200 | 5,0 mg | 7,5 g |
| | 755,0 mg | 1132,5 g |

Herstellung: wie Beispiel 1.

Eigenschaften

Freifliessend, geschmacklos.

Freigabeeigenschaften von Kaliumchlorid (Vanderkamp Zerfallstester, in Wasser von 37°C):

| | |
|---|---|
| nach 1 Stunde | ca. 38% |
| nach 2 Stunden | ca. 74% |
| nach 3 Stunden | ca. 96%. |

Abfüllung in Gelatinekapseln: wie Beispiel 1.

Beispiel 3:

| Zusammensetzung: | Dosis mg/Ansatz g |
|---|---|
| Pirprofenkristalle (0,5 mm) | 600.0 mg/g |
| Eudragit® E30D Feststoff | 24.0 mg/g (80 g Dispersion) |
| Aquacoat® ECD-30 Feststoff | 6.0 mg/g (20 g Dispersion) |
| Aerosil® 200 | 5,0 mg/g |
| | 635.0 mg/g |

Herstellung:

1. Pirprofen vorlegen.
2. Eudragit® E 30 D und Aquacoat® ECD 30 unter schwachem Rühren vermischen.
3. 1. und 2. besprühen im Wirbelschichtgranulator Aeromatik Strea 1:
   - im Gleichstrom sprühen
   - Zulufttemperatur 35°C
   - Durchsatz ca. 8 g/Minute
4. Trocknen bei 35°C Zulufttemperatur (Wirbelschichttrockner Strea 1) ca. 10 Min.
5. Trockene umhüllte Körner mit Aerosil® 200 während 10 Minuten mischen.
6. Mischung 5. durch Sieb 2,0 mm sieben.

Beispiel 4: Runde oder unregelmässig geformte Partikel einer erstarrten Schmelze bestehend aus Diclofenac-Natrium (35 %), Cetylalkohol (62 %) und Lecithin (3 %) werden durch Ueberziehen mit einem Lackfilm bestehend aus Eudragit® E 30 D und Aquacoat® ECD 30 (Verhältnis Feststoffe 3T : 1T) nach Beispiel 2 umhüllt. Anschliessend werden diese umhüllten Partikel mit Aerosil® 200 vermischt, wodurch ein Zusammenbacken der umhüllten Partikel verhindert wird.

| Zusammensetzung: | Dosis mg/Ansatz g |
|---|---|
| geformte Partikel 1-1.5 mm | 285.7 mg/g |
| Eudragit® E 30 D Feststoff | 58.6 mg/g (197 g Dispersion) |
| Aquacoat® ECD 30 Feststoff | 20.0 mg/g (67 g Dispersion) |
| Aerosil® 200 | 0.7 mg/g |
| | 365.0 mg/g |

Patentansprüche

1. Körnige Arzneimittel-Retardform, enthaltend einen granulierten oder kristallinen Arzneimittelwirkstoff, umhüllt mit Wirkstoffabgabeverzögernden Hüllstoffen, dadurch gekennzeichnet, dass diese Hüllstoffe im wesentlichen aus einem homogenen Gemisch eines wasserunlöslichen, aber in Wasser dispergierbaren Polyacrylsäureesters und eines wasserunlöslichen aber in Wasser dispergierbaren Celluloseäthers bestehen.

2. Körnige Arzneimittel-Retardform gemäss Anspruch 1, dadurch gekennzeichnet, dass das Hüllstoffgemisch im wesentlichen aus Poly (H + meth)-acrylsäure(methyl + äthyl)estern und Aethylcellulose im Gewichtsverhältnis 2,5 : 1 bis 5 : 1 besteht.

3. Körnige Arzneimittel-Retardform gemäss Anspruch 2, dadurch gekennzeichnet, dass das Mischungsverhältnis der Hüllstoffe 3 : 1 beträgt.

4. Körnige Arzneimittel-Retardform gemäss Anspruch 1, dadurch gekennzeichnet, dass das Hüllstoffgemisch noch kollodiales Siliziumdioxid enthält.

5. Körnige Arzneimittel-Retardform gemäss Anspruch 1, dadurch gekennzeichnet, dass der Arzneimittelwirkstoff Kaliumchlorid im Grössenbereich 0.3 - 1.2 mm Durchmesser ist.

6. Körnige Arzneimittel-Retardform gemäss Anspruch 1, dadurch gekennzeichnet, dass der Arzneimittelwirkstoff Kaliumchlorid im Grössenbereich 0.5 - 1.2 mm Durchmesser ist.

7. Körnige Arzneimittel-Retardform gemäss Anspruch 1, dadurch gekennzeichnet, dass der Arzneimittelwirkstoff Pirprofen ist.

8. Körnige Arzneimittel-Retardform gemäss Anspruch 1, dadurch zeichnet, dass der Arzneimittelwirkstoff Diclofenac-Natrium ist.

0052075

Europäisches Patentamt

# EUROPÄISCHER RECHERCHENBERICHT

Nummer der Anmeldung
EP 81 81 0443

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| Y | DE - A - 2 336 218 (BYK GULDEN LOMBERG)<br>* Seite 4, Absatz 2 - Seite 6, Absatz 3; Seite 7, Tabelle, Verbindung Nr. 7; Seite 11, Absatz 2 - Seite 13, Absatz 2; Seite 19, Absatz 3 - Seite 20, Abbildung 1; Beispiel 2, Ansprüche 1-14 *<br>-- | 1-8 | A 61 K 9/32<br>9/36<br>9/52 |
| Y | US - A - 4 140 756 (MEAD JOHNSON)<br>* Spalte 2, Zeilen 3-9; Spalte 3, Zeile 58 - Spalte 4, Zeile 32; Spalte 5, Zeilen 1-9; Spalte 5, Zeilen 36-48; Spalte 6, Zeilen 35-51; Beispiele 1,2 *<br>-- | 1-8 | RECHERCHIERTE SACHGEBIETE (Int. Cl.³)<br>A 61 K 9/00 |
| A | FR - A - 2 390 959 (PRUGNAUD)<br>* Seite 3, Zeile 31 - Seite 4, Zeile 11; Seite 4, Zeile 30 - Seite 5, Zeile 29 *<br>-- | 1-3 | |
| A | EP - A - 0 008 780 (SHIN-ETSU)<br>* Seite 1, Absatz 1; Seite 2, Absatz 4, Seite 4, Absatz 3; Ansprüche 1,3,4,14 *<br>-- | 1,2 | KATEGORIE DER GENANNTEN DOKUMENTE<br>X: von besonderer Bedeutung allein betrachtet<br>Y: von besonderer Bedeutung in Verbindung mit einer anderen Veröffentlichung derselben Kategorie<br>A: technologischer Hintergrund<br>O: nichtschriftliche Offenbarung<br>P: Zwischenliteratur<br>T: der Erfindung zugrunde liegende Theorien oder Grundsätze<br>E: älteres Patentdokument, das jedoch erst am oder nach dem Anmeldedatum veröffentlicht worden ist<br>D: in der Anmeldung angeführtes Dokument<br>L: aus andern Gründen angeführtes Dokument |
| A | CHEMICAL ABSTRACTS, Band 82, Nr. 10, 10. März 1975, Zusammenfassung Nr. 64453a, Seite 469<br>Columbus, Ohio, US<br>K. LECHMANN et al.:"Use of aqueous dispersions of synthetic materials to coat drug forms" | ./. | |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 19-02-1982 | CONTET |

EPA form 1503.1 06.78

0052075
Nummer der Anmeldung
EP 81 81 0443

Europäisches Patentamt

# EUROPÄISCHER RECHERCHENBERICHT



| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| | & Rev. Quim. Ind. (Rio de Janeiro) 1974, 43 (503), 60-5; (504), 93-4; (505), 112, 114, 116, 118<br>* Zusammenfassung *<br>-- | | |
| A | UNLISTED DRUGS, Band 31, Nr. 4, April 1979<br>Verbindung Nr. 52C<br>* Pirprofen * | 7 | |
| | -- | | RECHERCHIERTE SACHGEBIETE (Int. Cl.³) |
| A | UNLISTED DRUGS, Band 31 Nr. 9, September 1979<br>Verbindung Nr. 133q<br>* Diclofenac Na *<br>---- | 8 | |